# EUROPEAN PATENT SPECIFICATION

(11) **EP 0 902 701 B1**
(45) Date of publication and mention of the grant of the patent: **07.08.2002**
(21) Application number: 97922925.9
(22) Date of filing: 30.04.1997
(51) Int. Cl.: A61M 15/00

(54) **DEVICE FOR SEPARATING A PARTICLE FRACTION IN AN INSPIRED AIR FLOW**
VORRICHTUNG ZUR TRENNUNG EINER PARTIKELFRAKTION IN EINEM EINZUATMENDEN LUFTSTROM
DISPOSITIF PERMETTANT DE SEPARER UNE FRACTION DE PARTICULE DANS UN FLUX D'AIR INSPIRE

(30) Priority: 02.05.1996 DE 19617546
(43) Date of publication of application: 24.03.1999
(73) Proprietor: Euro-Celtique S.A., 2330 Luxembourg (LU)
(72) Inventor: FLEISCHER, Wolfgang, D-55218 Ingelheim (DE)
(74) Representative: Hilleringmann, Jochen, Dipl.-Ing.
(86) International application number: EP9702220
(87) International publication number: WO9741910

(56) References cited:
- EP-A- 0 407 028
- EP-A- 0 608 176
- DE-A- 4 208 880
- GB-A- 2 248 400
- US-A- 4 049 200

## Description

The invention relates to a particle separator according to the preamble of claim 1.

In prophylactic and healing therapies of respiratory tract diseases which can be performed through resorption via the lung, use is made of inhalation devices by which the patient inhales a controlled-dosage aeresol, with or without a propellant gas, along with inhalable particles. For this purpose, the particles are taken in predetermined quantities (dosage) from a supply of a medicant by abrasion/scraping or by a different manner of detaching the particles, and then enter a carrier gas flow which together with the particles will be inhaled by the patient.

To reduce environmental damage, efforts are made to replace the dosage aeresol generators containing a propellant gas by generators which are free of propellant gas. Thus, presently, for generating the active-substance particles, use is made of powder generators provided to have an air flow passing therethrough which is generated by the patient when inhaling air. This air flow takes along the active-substance particles which have been abraded, scraped or otherwise detached from a medicant supply, so that an air flow containing active-substance particles will enter the oral and pharyngeal regions and the bronchi of the patient. Such inhalators with powder generators are known, for instance, from DE-A-40 27 390, DE-A-42 08 880, EP-A 0 407 028, EP-A-0 608 176, WO-A 93/24165, US-A-4,049,200 and GB-A-2 248 400.

For obtaining a good therapeutic effect, it is of advantage if the coarser particles of the flow of active-substance particles/air are retained and the patient will thus inhale primarily the smaller particles. Besides, depending on the respective composition of the active substance of the medicant supply, these coarser particles, which are deposited primarily in the oral and pharyngeal regions of the patient, can lead to uncomfortable side effects so that it is advisable also under this aspect to separate these coarser particles before the flow of active-substance particles/air reaches the oral and pharyngeal regions of the patient.

It is an object of the invention to improve inhalators of the above type to the effect that coarser are not inhaled by the patient.

For solving the above object, the invention proposes a particle separator for separating a particle fraction of an inhalable particle/air flow generated by inspired air, said particle separator being provided particularly to be mounted on a device for generating inhalable active-substance particles by surface treatment of a preferably solidified medicant supply and further comprising the features of claim 1. The subclaims relate to additional embodiments of the invention.

The carrier gas flowing through the housing of the particle separator is subjected to a plurality of deflections which cause the larger and heavier particles to be carried out of the carrier gas flow because of the large centrifugal force acting on them (fractionating particle separation). To deflect the carrier gas flow, the housing has arranged therein a plurality of partition walls with respectively at least one opening for restricting the cross section of the flow, the openings of respectively adjacent successive partition walls being displaced relative to each other. Particularly, the mutual displacement of the openings is selected in a manner providing for a helical carrier gas flow in the housing between the inlet and the outlet.

The particle separator according to the invention makes it possible, in a simple constructional manner, to retain and fractionate those particles of a flow of active-substance particles/air generated by air inhaled by the patient which are larger than a maximum allowable size, so that these particles will not enter the oral and pharyngeal regions of the patient. Thus, hardly any larger active-substance particles will become deposited in the oral and pharyngeal regions of the patient, thus reducing the extent of the side effects of the medicant. Instead, it is the smaller active-substance particles which are transported, by the air inhaled by the patient, all the way into the bronchi and lungs where an optimum therapeutic effect is obtained.

To keep the fractionating of particles substantially independent from the tidal volume and the rate of air flow, it is of advantage if - as observed over the whole flow path - the parameters of the flow, i.e. the velocity and/or the cross section of the flow as well as the flow volume, are varied, wherein this variation and these variations, respectively, can be performed regularly and irregularly, respectively, and/or continuously and discontinuously, respectively, and/or steadily and unsteadily, respectively. In this regard, the openings of the partition walls of the particle separator according to the invention have different sizes from one partition wall to the next partition wall with the size of the openings of the partition walls decreasing from the inlet to the outlet of the housing, particularly in a uniform manner. Such a change of the openings of the partition walls, which towards the outlet of the housing become ever smaller, causes the flow velocity to increase towards the outlet of the housing. The increase of the velocity results in an increase of the centrifugal forces acting on the particles, so that larger particles are separated in any event before the flow of active-substance particles/air has left the housing.

The particle separator of the invention can be mounted particularly onto the inhalators according to DE-A-40 27 390, EP-A-0 407 028 and WO-A-93/24165.

Tests performed with the inventive particle separator mounted onto the mouthpiece of the inhaltor according to WO-A 93/24165 have shown that - independent from the air flow volume, which as a matter of experience is between 300 cm³ and 1.5 dm³, and independent from the rate of air flow, which as a matter of experience is between 15 and 90 dm³ per minute - active-substance particles from a size of substantially 5.8 µm are separated, whereas the range of particle sizes below substantially 5.8 µm, i.e. particles of a size between 0.5 and substantially 5.8 µm, can be inhaled.

According to an advantageous embodiment of the invention, it is provided that said openings are formed as round holes in the partition walls, with the partition walls limiting the holes on all sides. The size of the portions of the partition walls bordering the openings defines the size of the baffle faces subjected to the onflow of the flow of active-substance particles/air. The displacement of the openings respecticvely to the regions of the partition walls adjacent the housing makes it possible to influence the size of the baffle faces. In a particularly advantageous configuration, the openings are delimited partially by the inner side of the housing, i.e. the openings are formed, as it were, as edge recesses of the partition walls.

By way of alternative or in addition to the variation of the size of the openings, it is provided according to another advantageous embodiment of the invention that the distance between respectively adjacent partition walls is different from one partition wall to the next one. In this regard, the distance of the partition walls is suitably reduced from the inlet towards the outlet of the housing, so that the deflections that the flow of active-substance particles/air undergoes within the housing of the particle separator, become ever smaller towards the outlet of the housing. Thereby, it is accomplished to a still higher extent that the larger active-substance particles, which have to be separated, are retained by the partition walls of the separator and thus do not issue from the outlet of the housing.

The ratio between the largest opening and the smallest opening of the partition walls is between 0.5 and 0.05 and particularly between 0.33 and 0.1. The sizes of the openings which are larger than the smallest opening and are smaller than the largest opening, vary in uniform or nonuniform steps. The ratio between the distances of adjacent partition walls is preferably between 1 and 1.5, the ratio between the largest distance and the smallest distance between partition walls ranging from 1 to 10.

Suitably, the housing of the particle absorber comprises two to seven and particularly three to five partition walls, wherein the best results have been obtained using a particle absorber with three partition walls.

An embodiment of the invention will be explained in greater detail hereunder with reference to the drawings. In the drawings -
- Fig. 1: is a perspective sectional view of an inhalator with powder generator for generating a flow of active-substance particles/air during inhalation of air through the inhalator,
- Fig. 2: is a longitudinal sectional view of an alternative particle-separator attachment unit for the powder generator of the inhalator according to Fig. 1, and
- Figs. 3 to 8: are sectional views of the housing of the particle separator according to Fig. 2, showing the partition walls of the separator and the size and arrangement of the openings in the flow direction.

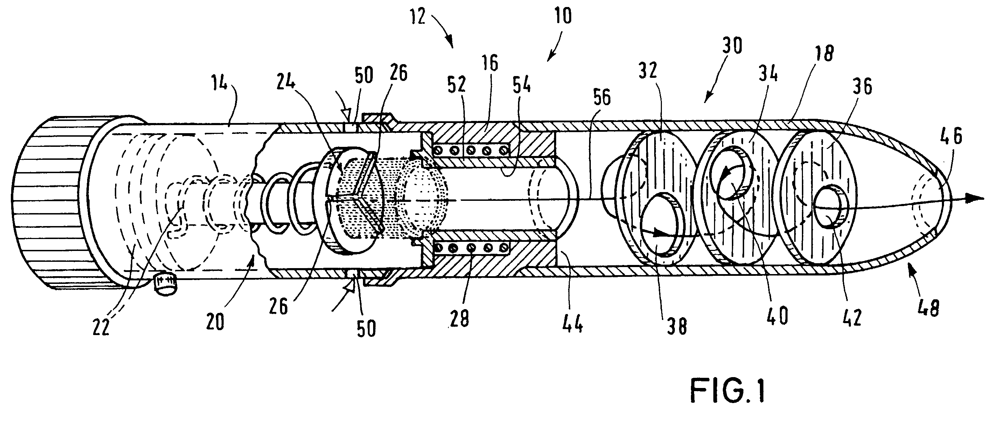

As shown in Fig. 1, the inhalator 10 comprises a three-part housing 12 consisting of three housing portions 14, 16 and 18 adapted to be mounted to each other. In housing portion 14, an automatic release device 20 is arranged for the dosed release of active-substance particles of a supply of a medicant. The release device 20 comprises a mechanical spring-wound motor 22 which can be wound up by hand and is arranged to rotate a end-milling cutter 24. The end-milling cutter 24 comprises cutting tools 25 abutting one end face of an annular solidified supply of a medicant 26. This medicant supply 26 is arranged in the central housing portion 16 of housing 12 and is pressed against the end-milling cutter 24 by a pressing spring 28. Mounted on the central housing portion 16 is the housing portion 18, forming part of a particle separator 30. Within the housing portion 18 (hereunder briefly referred to as "housing"), a plurality - in the instant embodiment, three - of partition or baffle walls 32,34,36 are arranged, each of them having an opening 38,40,42 formed therein. The housing 18 of particle separator 30 is further provided with an inlet 44 and an outlet 46 opposite thereto. In that region where the partition walls 32-36 are arranged within the housing, the housing 18 extends substantially in a cylindrical shape, whereas, starting from the partition wall closest to outlet 46 up to the outlet 46, the housing 18 has a conical shape forming a mouthpiece 48.

As evident from Fig. 1, the housing portion 14 containing the release device 20 is provided with air inlet openings 50 arranged level with the end-milling cutter 24. Between the air inlet openings 50 and the air outlet 46 of the particle separator, which air outlet 46 is also the air outlet of the inhalator 10, an air flow is generated when a patient inhales air through the air outlet 46. The air flow entering through the air inlet openings 50 passes through an intermediate space between the end-milling cutter 24 and the end face of medicant supply 26 from which particles are released by the action of the cutting tools 25 of end-milling cutter 24. From there, the air flow passes axially through the annular medicant supply and further through a press-on shell 52 which is in abutment on the medicant supply 26 and is pressed towards the end-milling cutter 24 by the spring 28 supported on a shoulder 54 of the central housing portion 16. The housing 18 of particle separator 30 is mounted to the central housing portion 16 so that the air leaving the central housing portion 16 enters the housing 18 through the inlet 44 of housing 18. As can be seen in Fig. 1, the openings 38, 40 and 42 of the partition walls 32,34,36 located behind each other in the flow direction 56, are arranged in a mutually displaced relationship, i.e. are arranged at positions rotated relative to each other about the longitudinal axis of housing 18. By this arrangement, the flow 46 is subjected to non-uniform deflections and thereby is caused to move in a substantially helical manner, thus following a curvilinear path. The openings 38-42 are reduced in size towards outlet 46; in other words, the opening 38 of the partition wall 32 of housing 18 closest to inlet 44 is largest, while the partition wall 36 closest to outlet 46 comprises the smallest opening 42. Further, the distance between adjacent partition walls varies towards outlet 46 of housing 18, which is also evident from Fig. 1. This means that the distance between the partition wall 32 facing towards the inlet 44 of housing 18 and the central partition wall 34, is larger than the distance between the central partition wall 34 and the partition wall 36 facing towards the outlet 46. The decrease of the size of the openings towards outlet 46 results in an increase of the flow velocity towards outlet 46. The reduction of the distances between adjacent partition walls 32,34,36 has the effect that the deflections that the flow is subjected to become narrower towards outlet 46. Both dimensional changes, i.e. the changes of the sizes of the openings and the changes of distances between the partition walls, lead to an optimum fractionating of the particles, which during rotation of the end-milling cutter 24 are removed from the medicant supply 26 and upon simultaneous suctional intake of air are taken along by the air flow. Notably, the centrifugal forces acting on the particles increase towards the outlet 46 of inhalator 10 because, on the one hand, the flow velocity is increased and, on the other hand, the flow is subjected to ever narrower deflections.

For experimental and testing purposes, a particle separator 30 of the type illustrated in Fig. 1 and described above was mounted to a device according to WO-A 93/24165. The housing 18 of particle separator 30 had a diameter of about 4 cm and an axial length of about 11 cm. The cross sections of the largest opening and the smallest opening 38,42 were 10 cm² and 1 cm², and particularly 6 cm² and 3 cm². The distance between the partition walls 32 and 34 was about 1.7 cm while the distance between the partition walls 34 and 36 was about 1.4 cm. The openings 38 and 40 were arranged at positions rotated relative to each other by about 90° around a longitudinal axis of the housing, while the openings 40 and 42 were arranged at positions rotated relative to each other by about 225°. All of the openings 38-42 substantially had the same smallest distance to housing 18. The distances between the inlet 44 of housing 18 and the first partition wall 32 in the flow direction 56, and between the last partition wall 36 in the flow direction 56 and the outlet 46 of housing 18 were respectively about 1/3 of the overall length of housing 18.

The above described particle separator 30 was used for the fractionating of particles from an air flow comprising particles with a Gaussian distribution of sizes between about 0.5 and 8 µm. With rates of air flow between 15 dm³ and 90 dm³ per minute, those particles which had a size larger than about 5,8 µm were in each case separated. In other words, only those particles which had a size between 0.5 and about 5,8 µm, passed through the separator 30. Thus, in each case, particles from a certain particle size could be separated irrespective of the rate of air flow. Under the therapeutic aspect, this offers the advantage that the side effects caused by the medicants due to depositions in the oral and pharyngeal regions can be considerably reduced.

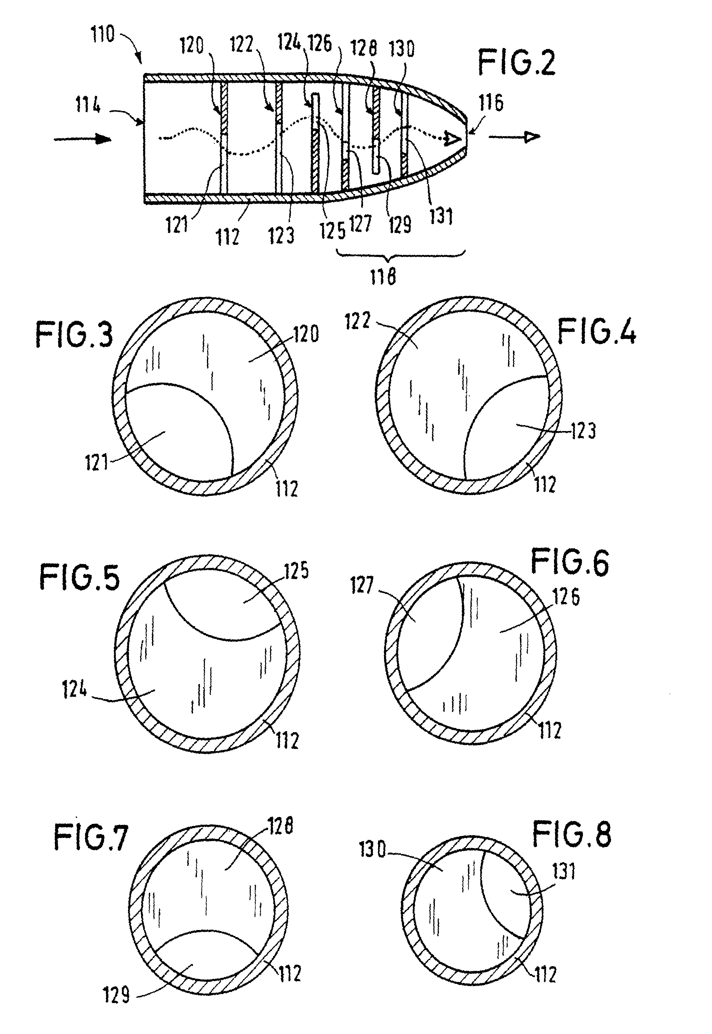

Fig. 2 is a perspective view of an alternative particle separator 110. The particle separator 110 comprises a substantially cylindrical housing 112 having an inlet opening 114 on one end and an outlet opening 116 on the other end. In its end portion 118 formed with the outlet opening 116, housing 112 is of a conically tapered shape.

Housing 112 can be mounted with its inlet opening 114 e.g. onto the central housing portion 16 of inhalator 10. Outlet opening 116 of housing 112 is adapted to have a mouthpiece connected thereto; alternatively, the end of housing 112 comprising the outlet opening 116 can be formed as a mouthpiece.

As evident from Fig. 2, housing 112 has arranged therein a plurality (in the instant embodiment, six) of partition walls 120 to 130. The distance between adjacent partition walls decreases from inlet opening 114 to outlet opening 116. Each of the partition walls 120 to 130 is provided with an opening 121 to 131 formed as an edge recess in partition walls 120 to 130. The size of the openings 121 to 131 decreases from inlet opening 114 to outlet opening 116 of housing 112 (cf. particularly Figs. 3 to 8). The openings 121 to 131 are arranged at positions rotated relative to each other (e.g. by 75° to 110°) about the longitudinal axis of particle separator 30. In this manner, the flow of the carrier gas and the particles passing through housing 112 is given a helical shape whose windings become narrower towards outlet opening 116 and whose flow velocity becomes larger towards outlet opening 116. Thereby, the heavier and larger particles are fractionated so that only the lighter, smaller particles will remain in the carrier gas flow, this effect being largely independent from the individual air flow rate of the patient which, as a matter of experience, is between 15 dm³ and 90 dm³ per minute.

## Claims

1. A particle separator for separating a particle fraction of an inhalable particle/air flow generated by inspired air, said particle separator being provided particularly to be mounted on a device for generating inhalable active-substance particles by release of said particles from a preferably solidified medicant supply, said particle separator comprising
- a housing (18;112) having said particle/air flow (56) flowing therethrough, said housing (18;112) comprising an inlet (44;114) and an outlet (46; 116), and
- a plurality of partition walls (32-36;120-130) arranged between said inlet (44;114) and said outlet (46;116) behind each other in the direction of the particle/air flow (56),
- each partition wall (32-36;120-130) having at least one opening (38-42;121-131) formed therein, and
- the openings (38-42;121-131) of adjacent partition walls (32-36;120-130) being arranged in a mutually displaced relationship resulting in a curvilinear portion of the flow path,
**characterized in that**
- the size of the openings (38-42;121-131) of the partition walls (32-36;120-130) is different from partition wall to partition wall, wherein the openings (38-42;121-131) of the partition walls (32-36;120-130) decrease in size from the inlet (44;114) to the outlet (46;116) of the housing (18;112).

2. The particle separator according to claim 1, **characterized in that** at least three partition walls (32-36;120-130) are provided.

3. The particle separator according to claim 1 or 2, **characterized in that** up to twenty, particularly up to ten and preferably up to seven partition walls (32-36;120-130) are provided.

4. The particle separator according to any one of claims 1 to 3, **characterized in that** the size of the openings (38-42;121-131) of the partition walls (32-36;120-130) decreases in a uniform manner from the inlet (44; 114) to the outlet (46;116) of the housing (18;112)

5. The particle separator according to any one of claims 1 to 4, **characterized in that** the distance between respectively adjacent partition walls (32-36;120-130) is different from partition wall to partition wall.

6. The particle separator according to claim 5, **characterized in that** the distance between respectively adjacent partition walls (32-36;120-130) decreases, particularly in a uniform manner, from the inlet (44;114) to the outlet (46; 116).

7. The particle separator according to any one of claims 1 to 6, **characterized in that** the openings (38-42;121-131) are formed by in particular round edge recesses of the partition walls (32-36;120-130), each of the openings (38-42;121-131) being delimited in a partial region of its edges by the housing (18;112).

8. The particle separator according to any one of claims 1 to 7, **characterized in that** the housing (18;112) is tapered, particularly conically, towards the outlet (46;116) to form a mouthpiece (48).

9. The particle separator according to any one of claims 1 to 8, **characterized in that** the openings (38-42;121-131) of the partition walls (32-36;120-130) are arranged at positions rotated relative to each other.

10. A device for generating an inhalable active-substance-particle/air flow generated by inspired air, comprising
- a housing (12;14,16,18) having said particle/ air flow flowing therethrough along a flow path, said housing (12;14,16,18) comprising an air flow inlet (50) and an active-substance particle/air flow outlet (46),
- a release device (20) for the release of active-substance particles from at least one partial surface of a supply of a medicant (26), said partial surface being arranged in the flow path behind said air flow inlet (50), and
- a particle separator (30) according to any one of the proceeding claims arranged in the housing (12;14,16,18) for separating particles, substantially active-substance particles, which are larger than a maximum size, said particle separator (30) being arranged in the flow path between said partial surface of the supply of the medicant (26) and said active-substance particle/air flow outlet (46).

11. The device according to claim 10, **characterized in that** the release device (20) is arranged for a dosed releasing of active-substance particles.

## Patentansprüche

1. Partikeltrennvorrichtung zum Trennen einer Partikelfraktion eines durch Ansaugluft erzeugten inhalierbaren Partikel-/Luftstroms, wobei die Partikeltrennvorrichtung insbesondere zum Anbringen an einer Vorrichtung zum Erzeugen von inhalierbaren Aktivsubstanzpartikeln durch Freisetzen der Partikel von einem zugeführten, vorzugsweise festen Medikament vorgesehen ist, wobei die Partikeltrennvorrichtung aufweist:
- ein Gehäuse (18;112), durch das der Partikel-/Luftstrom (56) strömt und das einen Einlass (44;114) und einen Auslass (46; 116) aufweist; und
- mehrere Trennwände (32-36;120-130), die zwischen dem Einlass (44;114) und dem Auslass (46;116) in Richtung des Partikel-/Luftstroms (56) gesehen hintereinander angeordnet sind,
- wobei jede Trennwand (32-36;120-130) mindestens eine darin ausgebildete Öffnung (38-42;121-131) aufweist, und
- die Öffnungen (38-42;121-131) benachbarter Trennwände (32-36;120-130) voneinander versetzt angeordnet sind, was zu einem krummlinigen Teil des Strömungspfads führt;
**dadurch gekennzeichnet, dass**
- die Größe der Öffnungen (38-42;121-131) der Trennwände (32-36;120-130) von Trennwand zu Trennwand unterschiedlich ist,
wobei die Größe der Öffnungen (38-42;121-131) der Trennwände (32-36;120-130) vom Einlass (44;114) zum Auslass (46;116) des Gehäuses (18;112) abnimmt.

2. Partikeltrennvorrichtung nach Anspruch 1, **dadurch gekennzeichnet, dass** mindestens drei Trennwände (32-36;120-130) vorgesehen sind.

3. Partikeltrennvorrichtung nach Anspruch 1 oder 2, **dadurch gekennzeichnet, dass** bis zu zwanzig, insbesondere bis zu zehn und vorzugsweise bis zu sieben Trennwände (32-36;120-130) vorgesehen sind.

4. Partikeltrennvorrichtung nach einem der Ansprüche 1 bis 3, **dadurch gekennzeichnet, dass** die Größe der Öffnungen (38-42;121-131) der Trennwände (32-36;120-130) vom Einlass (44;114) zum Auslass (46; 116) des Gehäuses (18;112) gleichmäßig abnimmt.

5. Partikeltrennvorrichtung nach einem der Ansprüche 1 bis 4, **dadurch gekennzeichnet, dass** der Abstand zwischen jeweils benachbarten Trennwänden (32-36;120-130) von Trennwand zu Trennwand unterschiedlich ist.

6. Partikeltrennvorrichtung nach Anspruch 5, **dadurch gekennzeichnet, dass** der Abstand zwischen jeweils benachbarten Trennwänden (32-36;120-130) vom Einlass (44;114) zum Auslass (46;116) insbesondere gleichmäßig abnimmt.

7. Partikeltrennvorrichtung nach einem der Ansprüche 1 bis 6, **dadurch gekennzeichnet, dass** die Öffnungen (38-42;121-131) von insbesondere runden Randausnehmungen der Trennwände (32-36;120-130) gebildet sind, wobei jede Öffnung (38-42;121-131) in einem Teilbereich ihrer Ränder von dem Gehäuse (18;112) begrenzt wird.

8. Partikeltrennvorrichtung nach einem der Ansprüche 1 bis 7, **dadurch gekennzeichnet, dass** sich das Gehäuse (18;112) in Richtung des Auslasses (46;116) insbesondere konisch verjüngt, um ein Mundstück (48) zu bilden.

9. Partikeltrennvorrichtung nach einem der Ansprüche 1 bis 8, **dadurch gekennzeichnet, dass** die Öffnungen (38-42;121-131) der Trennwände (32-36; 120-130) in gegeneinander versetzten Positionen angeordnet sind.

10. Vorrichtung zum Erzeugen eines durch Ansaugluft erzeugten inhalierbaren Aktlvsubstanzpartikel-/Luftstroms, mit:
- einem Gehäuse (12;14,16,18), durch das der Partikel-/Luftstrom einen Strömungspfad entlang strömt und das einen Luftstromeinlass (50) und einen Aktivsubstanzpartlkel-/Luftstromauslass (46) aufweist,
- einer Freisetzvorrichtung (20) zum Freisetzen von Aktivsubstanzpartikeln von mindestens einer Teilfläche eines zugeführten Medikaments (26), wobei die Teilfläche in dem Strömungspfad hinter dem Luftstromeinlass (50) vorgesehen ist, und
- einer Partikeltrennvorrichtung (30) nach einem der vorhergehenden Ansprüche, die zum Trennen von Partikeln, im wesentlichen Aktivsubstanzpartikeln, die eine Maximalgröße überschreiten, in dem Gehäuse (12;14,16,18) angeordnet ist, wobei die Partikeltrennvorrichtung (30) in dem Strömungspfad zwischen der Teilfläche des zugeführten Medikaments (26) und dem Aktivsubstanzpartlkel-/Luftstromauslass (46) angeordnet ist.

11. Vorrichtung nach Anspruch 10, **dadurch gekennzeichnet, dass** die Freisetzvorrichtung (20) für dosiertes Freisetzen von Aktivsubstanzpartikeln vorgesehen ist.

## Revendications

1. Séparateur de particule pour séparer une fraction de particule d'un flux de particules/air pouvant être inhalé, produit par de l'air inspiré, ledit séparateur de particule étant prévu en particulier pour être monté sur un dispositif pour produire des particules de substances actives pouvant être inhalées par libération desdites particules à partir d'une réserve de médicament de préférence solidifiée, ledit séparateur de particule comprenant
- un boîtier (18; 112) traversé par ledit flux de particules/air (56), ledit boîtier (18; 112) comprenant une entrée (44; 114) et une sortie (46; 116), et
- une pluralité de parois de séparation (32 à 36; 120 à 130) agencées entre ladite entrée (44; 114) et ladite sortie (46; 116) les unes derrière les autres dans la direction du flux de particules/air (56),
- chaque paroi de séparation (32 à 36; 120 à 130) comportant au moins une ouverture (38 à 42; 121 à 131) formée dans celle-ci, et
- les ouvertures (38 à 42; 121 à 131) des parois de séparations adjacentes (32 à 36; 120 à 130) étant agencées selon une relation mutuellement séparée résultant en une partie curviligne de la trajectoire de flux,
**caractérisé en ce que**
- la taille des ouvertures (38 à 42; 121 à 131) des parois de séparation (32 à 36; 120 à 130) est différente d'une paroi de séparation à une autre paroi de séparation, les ouvertures (38 à 42; 121 à 131) des parois de séparation (32 à 36; 120 à 130) ayant une taille qui diminue de l'entrée (44; 114) à la sortie (46; 116) du boîtier (18; 112).

2. Séparateur de particules selon la revendication 1, **caractérisé en ce qu'**au moins trois parois de séparation (32 à 36; 120 à 130) sont prévues.

3. Séparateur de particules selon la revendication 1 ou 2, **caractérisé en ce que** jusqu'à vingt, en particulier jusqu'à dix et de préférences jusqu'à sept parois de séparation (32 à 36; 120 à 130) sont prévues.

4. Séparateur de particules selon l'une quelconque des revendications 1 à 3, **caractérisé en ce que** la taille des ouvertures (38 à 42; 121 à 131) des parois de séparation (32 à 36; 120 à 130) diminue d'une manière uniforme de l'entrée (44; 114) à la sortie (46; 116) du boîtier (18; 112).

5. Séparateur de particules selon l'une quelconque des revendications 1 à 4, **caractérisé en ce que** la distance entre des parois de séparation respectivement adjacentes (32 à 36; 120 à 130) est différente d'une paroi de séparation à une autre paroi de séparation.

6. Séparateur de particules selon la revendication 5, **caractérisé en ce que** la distance entre des parois de séparation respectivement adjacentes (32 à 36; 120 à 130) diminue, en particulier d'une manière uniforme, de l'entrée (44; 114) à la sortie (46; 116).

7. Séparateur de particules selon l'une quelconque des revendications 1 à 6, **caractérisé en ce que** les ouvertures (38 à 42; 121 à 131) sont formées en particulier par des parties de bord rondes en retrait des parois de séparation (32 à 36; 120 à 130), chacune des ouvertures (38 à 42; 121 à 131) étant délimitée dans une région partielle de ses bords par le boîtier (18; 112).

8. Séparateur de particules selon l'une quelconque des revendications 1 à 7, **caractérisé en ce que** le boîtier (18; 112) est effilé, en particulier de façon conique, vers la sortie (46; 116) pour former une embouchure (48).

9. Séparateur de particules selon l'une quelconque des revendications 1 à 8, **caractérisé en ce que** les ouvertures (38 à 42; 121 à 131) des parois de séparation (32 à 36; 120 à 130) sont agencées dans des positions tournées les unes par rapport aux autres.

10. Dispositif pour produire un flux de particules de substances actives/air pouvant être inhalé produit par de l'air inspiré, comprenant
- un boîtier (12; 14, 16, 18) traversé par ledit flux de particules/air sur un trajet de flux, ledit boîtier (12; 14, 16, 18) comprenant une entrée de flux d'air (50) et une sortie de flux de particules de substances actives/air (46),
- un dispositif de libération (20) pour la libération des particules de substances actives depuis au moins une surface partielle d'une réserve de médicament (26), ladite surface partielle étant agencée dans le trajet de flux derrière ladite entrée de flux d'air (50), et
- un séparateur de particules (30) selon l'une quelconque des revendications précédentes, agencé dans le boîtier (12; 14, 16, 18) pour séparer des particules, les particules de substances sensiblement actives qui sont plus grandes qu'une taille minimale, ledit séparateur de particules (30) étant agencé dans le trajet de flux entre ladite surface partielle de la réserve de médicament (26) et ladite sortie de flux de particules de substances actives/air (46).

11. Dispositif selon la revendication 10, **caractérisé en ce que** le dispositif de libération (20) est agencé pour une libération fermée des particules de substances actives.
